# EUROPÄISCHE PATENTSCHRIFT

(11) **EP 0 419 950 B1**
(45) Veröffentlichungstag und Bekanntmachung des Hinweises auf die Patenterteilung: **30.11.1994**
(21) Anmeldenummer: 90117602.4
(22) Anmeldetag: 12.09.1990
(51) Int. Cl.: A61B 6/00

(54) **Röntgenuntersuchungsgerät**
X-ray examination apparatus
Appareil d'examen par radiographie

(30) Priorität: 27.09.1989 SE 8903175
(43) Veröffentlichungstag der Anmeldung: 03.04.1991
(73) Patentinhaber: Siemens-Elema AB, 171 95 Solna 1 (SE); SIEMENS AKTIENGESELLSCHAFT, 80333 München (DE)
(72) Erfinder: Larsson, Sten, S-162 40 Vällingby (SE)

(56) Entgegenhaltungen:
- EP-A- 0 345 138
- DE-A- 3 218 301
- FR-A- 2 406 426
- FR-A- 2 645 007

## Beschreibung

Die Erfindung betrifft ein Röntgenuntersuchungsgerät mit einem durchgehenden starren, gebogenen Träger, an dessen einem Ende eine Strahlungsquelle und an dessen anderem Ende ein Bildschichtträger aufeinander ausgerichtet befestigt sind und mit einer Halterung für den Träger, wobei die Strahlungsquelle und der Bildschichtträger über seitlich herausragende Ausleger mit dem Träger verbunden sind.

Ein Röntgenuntersuchungsgerät dieser Art ist durch die DE-OS 26 08 461 bekannt.

Bei Röntgenuntersuchungen z.B. in Verbindung mit einer Katheterisierung ist es von grosser Bedeutung, dass der Arzt eine gute Zugänglichkeit zum Patienten hat. Bei derartigen Untersuchungen ist meistens der Untersuchungstisch und das Röntgenuntersuchungsstativ parallel zueinander verschiebbar, so dass die Strahlungsquelle und der Bildschichtträger zwischen Kopf- und Fussende des Untersuchungstisches verschiebbar ist. Die Zugänglichkeit zum Patienten ist nicht immer gegeben, da das Stativ mit dem Träger, der z.B. C-, V- oder U-förmig sein kann, bei gewissen Untersuchungen im Wege sein kann. Das kann der Fall sein, wenn das Katheter in die Leiste eingeführt und nach unten in ein Bein hinuntergeschoben wird. Der Arzt kann dabei an derselben Seite stehen, an der das Stativ angebracht ist. Wenn die Strahlungsquelle und der Bildschichtträger dem Weg des Katheters bzw. des Kontrastmittels folgen und diese abbilden, wird sich das Stativ zum Arzt hin bewegen.

Der Erfindung liegt die Aufgabe zugrunde, ein Röntgenuntersuchungsgerät der eingangs genannten Art zu schaffen, das mit einfachen Mitteln eine sehr gute Zugänglichkeit zum Patienten bei verschiedenen Arten von Untersuchungen erlaubt.

Diese Aufgabe ist erfindungsgemäss dadurch gelöst, dass die Ausleger mit dem Träger um je eine Achse drehbar verbunden sind. Hierdurch kann die Dezentrierung der Strahlungsquelle und des Bildschichtträgers gegenüber dem Träger in eine spiegelverkehrte, dezentrierte Lage überführt werden. Auf diese Weise kann der Arzt selbst entscheiden, auf welcher Seite des Trägers sich die Strahlungsquelle bzw. der Bildschichtträger befinden soll. Ein weiterer Vorteil der Erfindung besteht bei einem Röntgenuntersuchungsgerät, dass als Boden- oder Deckenstativ ausgebildet ist, das parallel mit dem Untersuchungstisch bewegt wird darin, dass es eine kürzere Strecke als frühere Stative transportiert werden muss und dadurch weniger Platz in Anspruch nimmt, da die Strahlungsquelle und der Bildschichtträger gemäss der Erfindung eine grössere Reichweite haben.

In der FR-A-2 406 426 ist ein Röntgenuntersuchungsgerät beschrieben, das zwei teleskopartigen Deckenstative umfasst, wobei das eine Stativ eine Röntgenröhre und das andere Stativ eine Röntgenaufnahmeeinrichtung trägt. Die Stative, die an jeweils einer Laufschiene unabhängig voneinander verschiebbar gelagert und durch ihren teleskopartigen Aufbau höhenverstellbar sind, sind ausserdem an einem gemeinsam an der Decke befestigten Drehgestell aufgehängt. Die Röntgenröhre und die Aufnahmeeinrichtung sind um jeweils eine horizontal angebrachte Achse drehbar gehaltert. Die Achsen sind ihrerseits mit vertikal angebrachten Geräteträgern, die um ihre eigene Achsen drehbar sind, fest verbunden. Dieses Röntgenuntersuchungsgerät ist im Aufbau verhältnismässig kompliziert, da die Röntgenröhre und die Röntgenaufnahmeeinrichtung an zwei verschiedene Stative angebracht sind und dadurch in fast allen Stativlagen gegeneinander ausgerichtet werden müssen.

In einer vorteilhaften Ausbildung der Erfindung wird vorgeschlagen, dass der Bildschichtträger bzw. die Strahlungsquelle um jeweils eine zweite Achse derart drehbar sind, dass der Bildschichtträger bzw. die Strahlungsquelle in bestimmten Lagen des Auslegers gegeneinander gerichtet werden können. Eine solche zweite Achse kann mit Vorteil verwendet werden, wenn der Träger z.B. V-förmig ist und wenn die Achsen der Ausleger nicht achsfluchtend angeordnet oder parallel miteinander sind.

Eine weitere günstige Ausgestaltung ergibt sich, wenn der Bildschichtträger bzw. die Strahlungsquelle mit Hilfe von jeweils einer Antriebsvorrichtung drehbar ist. So kann die gegenseitige Ausrichtung des Bildschichtträgers und der Strahlungsquelle automatisch erfolgen.

In einer weiteren Ausgestaltung der Erfindung wird vorgeschlagen, daß der Ausleger mit Hilfe von Antriebsvorrichtungen synchron drehbar sind. Die Strahlungsquelle und der Bildschichtträger können auf diese Weise rasch in eine gewünschte Lage gebracht werden.

Weitere Vorteile und Einzelheiten ergeben sich aus den Unteransprüchen.

Die Erfindung ist nachfolgend anhand von zwei in den Zeichnungen dargestellten Ausführungsbeispielen näher erläutert. Es zeigen:
- FIG 1: eine perspektivische Ansicht eines Röntgenuntersuchungsgerätes nach der Erfindung und
- FIG 2: eine weitere Ausführungsform des Röntgenuntersuchungsgerätes nach der Erfindung.

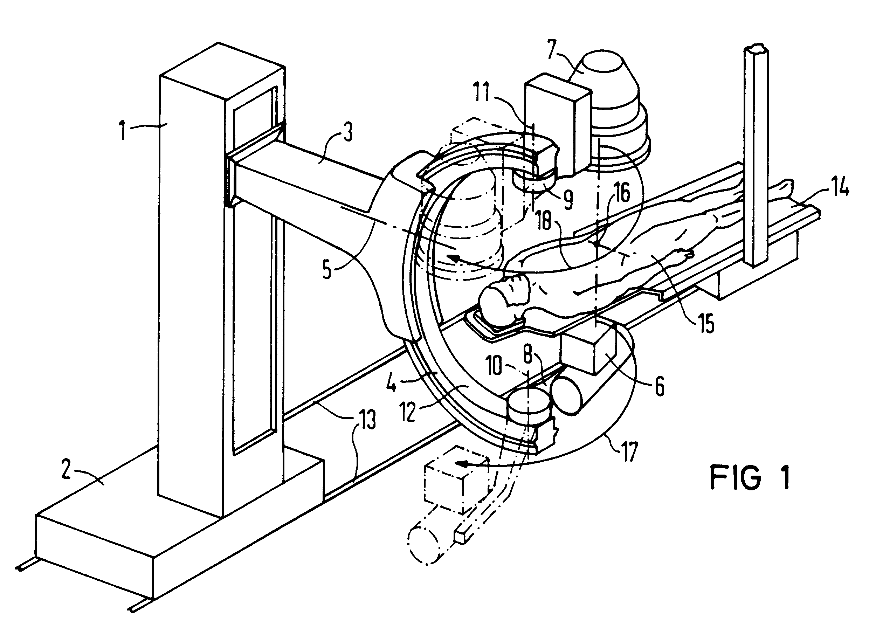

In der FIG 1 ist ein Röntgenuntersuchungsgerät mit einer Stativsäule (1) gezeigt, die auf einem Stativwagen (2) angebracht ist. An der Stativsäule (1) befindet sich eine Halterung (3) für einen halbkreisförmig gebogenen Träger (4), der an der Stativsäule (1) längsverschiebbar angebracht ist. Die Halterung (3) und damit auch der Träger (4) sind um eine horizontale Achse (5) schwenkbar gelagert. An dem einen Ende des Trägers (4) ist eine Röntgenröhre (6) und an dem anderen Ende ein Röntgenbildverstärker (7) aufeinander ausgerichtet befestigt, wobei diese über jeweils einen herausragenden Ausleger (8,9) mit dem Träger (4) verbunden sind. Außerdem sind die Ausleger (8,9) mit der Innenoberfläche (12) des Trägers (4) um jeweils eine Achse (10,11) drehbar verbunden, die achsfluchtend zueinander liegen. Der Träger (4) ist auch längs seiner Biegung verschiebbar gelagert. Die Halterung (3) läßt die Innenoberfläche (12) des Trägers über ihre gesamte Länge frei. Der Stativwagen (2) mit der Stativsäule (1) ist auf Bodenschienen (13) fahrbar.

Die Röntgenröhre (6) und der Röntgenbildverstärker (7) können durch die beschriebenen Bewegungsabläufe des Stativwagens (2), der Halterung (3) und des Trägers (4), die mit Hilfe einer Regeleinrichtung, wie beispielhaft die in der DE-OS 34 13 348 gezeigt und näher beschrieben, dazu gebracht werden, sich in einem sphärischen Muster um ein fiktives Isozentrum zu bewegen, z.B. um den Punkt (16) eines auf einem Untersuchungstisch (14) ruhenden Patienten (15). Außerdem kann der Stativwagen (2) mit der Stativsäule (1) parallel und entlang des Untersuchungstisches verschoben werden.

Dadurch, daß die Röntgenröhre (6) und der Röntgenbildverstärker (7) durch die Ausleger (8,9) um die Achsen (10,11) z.B. 180° gedreht werden können, wie es die Pfeile (17,18) andeuten, kann die Dezentrierung der Röntgenröhre (6) und des Röntgenbildverstärkers (7) gegenüber dem Träger spiegelverkehrt erfolgen. Diese weitere Lage der Röntgenröhre (6) und des Bildverstärkers (7) wird mit strichpunktierten Konturen gezeigt. Die Röntgenröhre (6) und der Bildverstärker (7) können auch in dieser Lage mit Hilfe der erwähnten Regeleinrichtung in einem sphärischen Bewegungsmuster um ein fiktives Isozentrum geschwenkt werden. Durch diese Möglichkeit zum Schwenken der Röntgenröhre (6) bzw. des Bildverstärkers (7), das vorzugsweise mit Hilfe von nicht dargestellten Antriebsvorrichtungen synchron erfolgen kann, kann der Arzt selbst entscheiden, in welcher Position die Röntgenröhre (6) bzw. der Röntgenbildverstärker (7) sich befinden sollen.

Bei der beschriebenen Schwenkung der Röntgenröhre (6) und des Röntgenbildverstärkers (7) von der einen zu der anderen Seite des Trägers (4) können diese in einer beliebigen Position arretiert werden, wobei eine Röntgenuntersuchung bei Bedarf durchgeführt werden kann.

Die Wellen (10,11) der Ausleger (8,9) können auch parallel zueinander angeordnet sein. Bei einer solchen Ausführungsform sollte jedoch die Röntgenröhre (6) bzw. der Röntgenbildverstärker (7) und/oder die Ausleger (8,9) mit Hilfe von nicht dargestellten Antriebsvorrichtungen derart gesteuert werden, daß die Röntgenröhre (6) und der Röntgenbildverstärker (7) in jeder Lage aufeinander ausgerichtet sind.

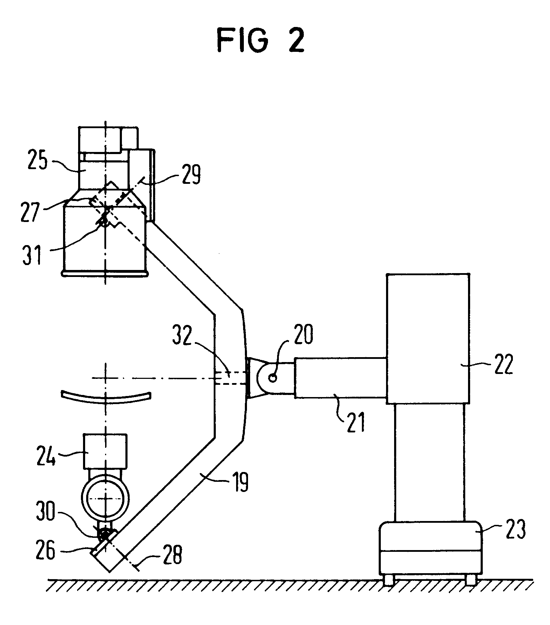

In der FIG 2 ist eine weitere Ausführungsform eines Röntgenuntersuchungsgerätes mit einem U- oder V-förmigen Träger (19) gezeigt, der eine Röntgenröhre (24) und einen Röntgenbildverstärker (25) trägt und der über einen Verbindungspunkt mit einer Halterung (21) verbunden ist. Die Halterung (21) ist ihrerseits an einer Stativsäule (22), die an einem Stativwagen (23) angebracht ist, in der Höhe verstellbar befestigt. Der Träger (19) ist um eine horizontale Welle (32) schwenkbar. Ein Röntgenuntersuchungsgerät dieser Art ist in der DE-OS 34 13 348 gezeigt und näher beschrieben. Bei der in dieser Figur dargestellten Ausführungsform ist die Röntgenröhre (24) und der Röntgenbildverstärker (25) mit dem Träger herausragenden Ausleger (26,27), die an dem Träger (19) um jeweils eine Welle (28,29) schwenkbar befestigt sind, verbunden. Da die Wellen (28,29) nicht achsfluchtend zueinander liegen und auch nicht parallel zueinander angebracht sind, ist die Röntgenröhre (24) bzw. der Röntgenbildverstärker (25) um jeweils eine zweite Welle (30, 31) derart drehbar, dass die Röntgenröhre bzw. der Röntgenbildverstärker (25) in bestimmten Lagen der Ausleger (26, 27) aufeinander gerichtet werden können. Die Wellen (30, 31) sind mit Hilfe von jeweils einer Antriebsvorrichtung, die mit der in der erwähnten DE-OS 34 13 348 beschriebenen Regeleinrichtung ergänzt werden können, drehbar.

### Bezugszeichenliste

- 1, 22: Stativsäule
- 2, 23: Stativwagen
- 3, 21: Halterung
- 4, 19: Träger
- 5,10,11,28,29,30,31,32: Welle
- 6, 24: Röntgenröhre Strahlungsquelle
- 7,25: Röntgenbildverstärker Bildschichtträger
- 8, 9, 26 ,27: Ausleger
- 12: Innenoberfläche
- 13: Schiene
- 14: Untersuchungstisch
- 15: Patient
- 16: Punkt
- 17, 18: Pfeil
- 20: Verbindungspunkt

## Patentansprüche

1. Röntgenuntersuchungsgerät mit einem durchgehenden starren, gebogenen Träger (4,19) an dessen einem Ende eine Strahlungsquelle (6,24) und an dessen anderem Ende ein Bildschichtträger (7,25) aufeinander ausgerichtet befestigt sind und mit einer Halterung (3,21) für den Träger (4,19), wobei die Strahlungsquelle (6,24) und der Bildschichtträger (7,25) über seitlich herausragende Ausleger (8,9,26, 27) mit dem Träger (4,19) verbunden sind, **dadurch gekennzeichnet,** dass die Ausleger (8,9,26,27) mit dem Träger (4,19) um jeweils eine Achse (10,11,28,29) drehbar verbunden sind.

2. Röntgenuntersuchungsgerät nach Anspruch 1, **dadurch gekennzeichnet,** dass die beiden Achsen (10,11) achsfluchtend zueinander liegen.

3. Röntgenuntersuchungsgerät nach Anspruch 1, **dadurch gekennzeichnet,** dass die Achsen (10,11) parallel zueinander angeordnet sind, wobei die Strahlungsquelle (24) und der Bildschichtträger (25) mittels Antriebsvorrichtungen gesteuert werden.

4. Röntgenuntersuchungsgerät nach einem der Ansprüche 1 bis 3, **dadurch gekennzeichnet,** dass der Bildschichtträger (25) bzw. die Strahlungsquelle (24) um jeweils eine zweite Achse (30,31) derart drehbar sind, dass der Bildschichtträger (25) bzw. die Strahlungsquelle (24) in bestimmten Lagen des Auslegers (26,27) aufeinander gerichtet werden können.

5. Röntgenuntersuchungsgerät nach Anspruch 4, **dadurch gekennzeichnet,** dass der Bildschichtträger (25) bzw. die Strahlungsquelle (24) mit Hilfe von jeweils einer Antriebvorrichtung drehbar sind.

6. Röntgenuntersuchungsgerät nach einem der Ansprüche 1 bis 5, **dadurch gekennzeichnet,** dass der Ausleger (8,9, 26,27) mit Hilfe von Antriebsvorrichtungen synchron drehbar sind.

## Claims

1. X-ray examination apparatus having a continuous rigid, curved carrier (4, 19), to one end of which a radiation source (6, 24) and to the other end of which an image medium support (7, 25) are secured so as to be aligned with one another, and having a mounting (3, 21) for the carrier (4, 19), the radiation source (6, 24) and the image medium support (7, 25) being connected to the carrier (4, 19) via laterally projecting arms (8, 9, 26, 27), characterized in that the arms (8, 9, 26, 27) are connected to the carrier (4, 19) so as to be rotatable about a respective axis (10, 11, 28, 29).

2. X-ray examination apparatus according to Claim 1, characterized in that the two axes (10, 11) are situated in axial alignment with one another.

3. X-ray examination apparatus according to Claim 1, characterized in that the axes (10, 11) are disposed parallel to one another, the radiation source (24) and the image medium support (25) being controlled by means of drive devices.

4. X-ray examination apparatus according to one of Claims 1 to 3, characterized in that the image medium support (25) and the radiation source (24) respectively are rotatable about a respective second axis (30, 31) in such a manner that the image medium support (25) and the radiation source (24) respectively can be directed at one another in specified positions of the arm (26, 27).

5. X-ray examination apparatus according to Claim 4, characterized in that the image medium support (25) and the radiation source (24) respectively are rotatable with the aid of a respective drive device.

6. X-ray examination apparatus according to one of Claims 1 to 5, characterized in that the arms (8, 9, 26, 27) are rotatable synchronously with the aid of drive devices.

## Revendications

1. Appareil d'examen par radiographie, comportant un support (4,19) coudé, rigide; d'un seul tenant, sur une extrémité duquel est fixée une source de rayonnement (6,24) et sur l'autre extrémité duquel est fixé un support de couche d'image (7,25), la source de rayonnement et le support de couche d'image étant alignés l'un sur l'autre, et un dispositif de fixation (3,21) du support (4,19), la source de rayonnement (6,24) et le support de couche d'image (17,25) étant reliés au support (4,19) par l'intermédiaire de bras en porte-à-faux (8,9,26,27) qui font saillie latéralement, caractérisé par le fait que les bras en porte-à-faux (8,9,26,27) sont reliés au support (4,19) de manière à pouvoir tourner respectivement autour d'un axe (10,11,28,29).

2. Appareil d'examen par radiographie suivant la revendication 1, caractérisé par le fait que les deux axes (10,11) sont alignés l'un sur l'autre.

3. Appareil d'examen par radiographie suivant la revendication 1, caractérisé par le fait que les axes (10, 11) sont parallèles, la source de rayonnement (24) et le support de couche d'image (25) étant commandés au moyen de dispositifs d'entraînement (4).

4. Appareil d'examen par radiographie suivant l'une des revendications 1 à 3, caractérisé par le fait que le support de couche d'image (25) et la source de rayonnement (24) peuvent tourner autour de seconds axes (30,31) respectifs de telle sorte que le support de couche d'image (25) et la source de rayonnement (24) peuvent être alignés l'un sur l'autre lorsque le bras en porte-à-faux (26,27) est dans des positions déterminées.

5. Appareil d'examen par radiographie suivant la revendication 4, caractérisé par le fait que le support de couche d'image (25) et la source de rayonnement (24) peuvent être entraînés en rotation à l'aide d'un dispositif respectif d'entraînement.

6. Appareil d'examen par radiographie suivant l'une des revendications 1 à 5, caractérisé par le fait que les bras en porte-à-faux (8,9,26,27) peuvent être entraînés en rotation d'une manière synchrone à l'aide de dispositifs d'entraînement.
